(19) Europäisches Patentamt
European Patent Office
Office européen des brevets

(11) EP 4 037 641 B1

(12) EUROPEAN PATENT SPECIFICATION

(45) Date of publication and mention of the grant of the patent:
**26.04.2023 Bulletin 2023/17**

(21) Application number: **20775244.5**

(22) Date of filing: **16.09.2020**

(51) International Patent Classification (IPC):
***A61K 8/24*** (2006.01) ***A61K 8/90*** (2006.01)
***A61Q 11/00*** (2006.01) ***A61K 8/49*** (2006.01)

(52) Cooperative Patent Classification (CPC):
**A61Q 11/00; A61K 8/24; A61K 8/492; A61K 8/90**

(86) International application number:
**PCT/EP2020/075888**

(87) International publication number:
**WO 2021/063686 (08.04.2021 Gazette 2021/14)**

# (54) AN ORAL CARE COMPOSITION COMPRISING HEXAMETAPHOSPHATE AND A PIGMENT

MUNDPFLEGEZUSAMMENSETZUNG ENTHALTEND HEXAMETAPHOSPHATE UND EIN PIGMENT

COMPOSITION DE SOIN ORAL COMPRENANT DE L' HEXAMETAPHOSPHATE ET UN PIGMENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.10.2019 EP 19201370**

(43) Date of publication of application:
**10.08.2022 Bulletin 2022/32**

(73) Proprietors:
- **Unilever IP Holdings B.V.**
  **3013 AL Rotterdam (NL)**
  Designated Contracting States:
  **AL AT BE BG CH CZ DK EE ES FI FR GR HR HU IS LI LT LU LV MC MK NL NO PL PT RO SE SI SK SM**
- **Unilever Global IP Limited**
  **Wirral, Merseyside CH62 4ZD (GB)**
  Designated Contracting States:
  **CY DE GB IE IT MT RS TR**

(72) Inventors:
- **JOINER, Andrew**
  **Wirral Merseyside CH63 3JW (GB)**
- **PHILPOTTS, Carole, Jane**
  **Wirral Merseyside CH63 3JW (GB)**

(74) Representative: **Tansley, Sally Elizabeth**
**Unilever Patent Group**
**Bronland 14**
**6708 WH Wageningen (NL)**

(56) References cited:
**US-A- 4 370 314**
**US-A- 5 902 568**
**US-A1- 2007 140 986**
**US-A1- 2013 344 009**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 037 641 B1

## Description

### Field of the Invention

The present invention relates to an oral care composition for minimising staining of teeth.

### Background of the Invention

Long-lasting whitening of teeth is of considerable interest to consumers. Foods and drinks such as tea, coffee and wine may form dental stains by directly depositing chromogens on the tooth surface. Attraction of materials to the tooth surface plays a critical role in the deposition of extrinsic dental stain. The chromogens in these beverages that are responsible for causing dental stain are known as tannins and are composed of polyphenols such as catechins. These materials generate colour due to the presence of conjugated double bonds.

Traditional tooth whitening methods involve either peroxide bleaching from kit formats or abrasive stain removal from toothpaste formats. These particles are essentially insoluble particles that remove extrinsic stain from the surface of the tooth via abrasion when applied with a brush. The present invention relates to a novel oral care composition that delivers effective levels of stain protection and prolongs the effects of tooth whitening by preventing staining of teeth. The invention discloses a stain repellent coating having a synergistic blend of a hydrophilic-hydrophobic block co-polymer and $Ca^{2+}$ chelating agent (a phosphate salt) at a defined ratio. This helps to provide a protective stain coating on the tooth surface during the consumption of food/drinks post whitening treatments.

The block copolymers used in the present invention are what are generally known as Pluronics. These polymers, in general, have been known to be used in oral care. The phosphate salt used in the present invention is hexametaphosphate which has also been used in oral care.

US2014377194A (P&G) discloses an oral care composition containing (a) a zinc citrate; and (b) a surface-active organophosphate compound and it also relates to a method of preventing a stain from depositing on a tooth surface or other oral surfaces.

US2003124065A (P&G) discloses an oral care composition and methods for overall cleaning, whitening and preventing, reducing or removing surface deposited stains on natural teeth and dental prosthesis, the compositions comprising in an orally acceptable carrier at least 0.1% by weight of a water-soluble or water-dispersible copolymer prepared by copolymerizing one or a mixture of vinyl pyrrolidone (VP) monomers with one or a mixture of C1-C19 alkyl carboxylic acid (AC) C2-C12 alkenyl ester monomers; preferably, these compositions further comprise one or a mixture of other oral care agents selected from a water soluble alkali metal or ammonium tripolyphosphate in an amount at least about 0.5% by weight of the composition, an abrasive, preferably a precipitated silica abrasive, in an amount at least about 6% by weight of the composition and a bleaching agent in an amount at least about 0.1% by weight of the composition. US4370314B discloses an oral composition comprising Pluronic and Sodium hexametaphosphate.

It is thus an object of the present invention to provide for an oral care composition that minimizes staining of teeth.

### Description of the Invention

According to the first aspect of the present invention there is provided an oral care composition comprising:

(a) polymer having the structure

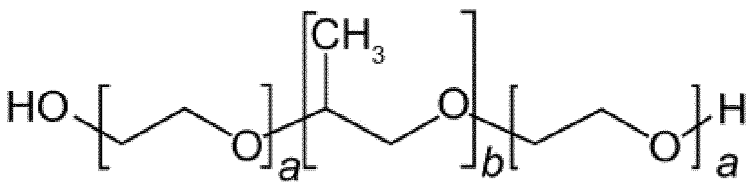

in which a is independently selected from 2 to 130 and b is 15 to 67 and the molecular weight of the polymer is from 1,700 to 15,000 Da.

(b) hexametaphosphate;

(c) blue covarine and and

(d) an orally acceptable base;

in which the weight ratio of the polymer to hexametaphosphate is from 1:5 to 5:1.

According to another aspect of the present invention there is provided a method of minimising or preventing

the staining of teeth comprising the steps of applying the composition of the invention described above on to a tooth.

A further aspect of the invention relates to cosmetic use of a composition according to any preceding claim to mitigate the staining of teeth.

## Detailed Description of the Invention

Except in the examples, or where otherwise explicitly indicated, all numbers in this description indicating amounts of material or conditions of reaction, physical properties of materials and/or use may optionally be understood as modified by the word "about".

All amounts are by weight of the final composition, unless otherwise specified.

It should be noted that in specifying any ranges of values, any particular upper value can be associated with any particular lower value.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

The present invention relates to an oral care composition comprising a polymer having the structure of compound of formula 1.

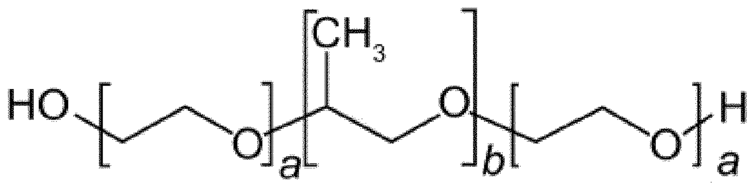

Compound of formula 1.

In the above compound, the molecular weight ratio of ethoxylate groups to the propoxylate groups is in the range of 0.1 to 3.0, preferably in the range of 0.1 to 2.5. The molecular weight of the polymer is in the range of from 1700 to 15000 Da, preferably in the range of 10000 to 13000 Da. The present invention preferably requires polymers, which contains both EO and PO (propoxylate) parts, with the above structure. The ethoxylate (EO) part of the polymer is to preferably be from 10 to 80% of the total polymer, more preferably from 10 to 60% of the total polymer. Commercially available polymers which are preferably used in the composition of the present invention include Pluronic L-61, Pluronic F88, Pluronic F77, Pluronic F108, or Pluronic 127, more preferably Pluronic 127. The polymer is preferably present in 0.05 to 4wt%, more preferably 0.1to 3wt%, most preferably from 0.1 to 2 wt% of the total composition

The composition of the invention includes a phosphate compound which is hexametaphosphate. Sodium hexametaphosphate has the structure as give below:

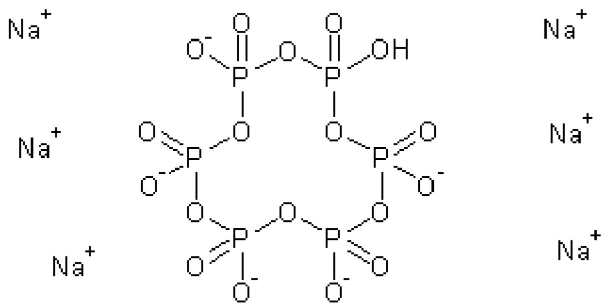

The hexametaphosphate is preferably included in 0.1 to 4%, more preferably 0.1 to 2.0% by weight of the total composition.

It is required as per the present invention that the weight ratio of polymer of formula 1 to hexametaphosphate is from 1:5 to 5:1, more preferably from 1:4 to 4:1. In a preferred aspect this ratio is from 1:3 to 3:1. It is particularly preferred if the weight ratio of polymer of formula 1 to hexametaphosphate is greater than 1:1.

It is preferred that the total amount of polymer and hexametaphosphate in the composition of the invention is from 0.1 to 5wt%, of the total composition.

The pigment used in the invention comprisesis Phthalocyanine Blue Pigment, CI No. 74160, blue covarine.

Preferably the weight ration of blue covarine to polymer (a) is from 5:100 to 1:1,000, more preferably from 3:200 to 1:500.

The amount of pigment in the total composition, particularly the pigments named above, in particular blue covarine is from 0.0005 wt% to 0.5 wt%, more preferably from 0.001 to 0.1 wt% most preferably from 0.007 to 0.014 wt%.

The percentage weights refer to the total amount of active present and exclude any carriers that may be present.

Compositions according to the invention can be in any orally acceptable form such as a toothpaste, mouthwash, spray, tablet, mask or serum, however sparys and toothpastes are preferred.

The composition of the invention comprises an orally acceptable base. The orally acceptable base preferably comprises, a humectant, water, volatile alcohol or combinations thereof. The orally acceptable base preferably makes up 96 to 99.8% by weight of the composition.

A composition according to the invention (will generally contain further ingredients to enhance performance and/or consumer acceptability, in addition to the ingredients specified above.

Compositions according to the invention may comprise a polymeric deposition aid. Preferably the composition comprises acid anhydride polymers, particularly preferred are co-polymers of maleic anhydride with methyl vinylether, in which the anhydride moiety may be in a partially or fully hydrolysed or alcoholysed form. Preferred copolymers include Gantrez(R) polymers such as:

Gantrez S-95: molecular weight 216,000; free acid;
Gantrez S-96: molecular weight 700,000; free acid;
Gantrez S-97: molecular weight 1,500,000; free acid; and
Gantrez MS-955: molecular weight 1,060,000; calcium/sodium salt.

Particularly preferred co-polymers of maleic acid and methyl vinylether have a molecular weight of 1,000,000 or greater and an especially preferred material is Gantrez S-97.

Compositions according to the invention may comprise water-soluble or sparingly water-soluble sources of metal salts. Preferred are zinc ions such as zinc chloride, zinc acetate, zinc gluconate, zinc sulphate, zinc fluoride, zinc citrate, zinc lactate, zinc oxide, zinc monoglycerolate, zinc tartrate, zinc pyrophosphate and zinc maleate; also preferred are stannous ions such as stannous fluoride and stannous chloride.

Compositions according to the invention may comprise oral care enzyme systems such as hydrogen peroxide producing enzyme systems (e.g. the oxidoreductase enzyme glucose oxidase), amyloglucosidase, dextranase and/or mutanase, (optionally in the presence of zinc ion providing compounds and/or 8- hydroxyquinoline derivatives), lactoperoxidase, lactoferrin, lysozyme and mixtures thereof.

Compositions of the invention may comprise fluoride sources such as sodium fluoride, stannous fluoride, sodium monofluorophosphate, zinc ammonium fluoride, tin ammonium fluoride, calcium fluoride, cobalt ammonium fluoride and mixtures thereof.

Preferably the composition has a pH at 20°C from 6 to 9.

The composition according the invention will comprise further ingredients which are common in the art, such as:

antimicrobial agents, e.g. chlorhexidine, sanguinarine extract, metronidazole,
quaternary ammonium compounds, such as cetylpyridinium chloride; bis-guanides,
such as chlorhexidine digluconate, hexetidine, octenidine, alexidine; and halogenated bisphenolic compounds, such as 2,2' methylenebis-(4-chloro-6-bromophenol);
anti-inflammatory agents such as ibuprofen, flurbiprofen, aspirin, indomethacin etc.; anti-caries agents such as sodium- and stannous fluoride, aminefluorides, sodium monofluorophosphate, sodium trimeta phosphate and casein;
plaque buffers such as urea, calcium lactate, calcium glycerophosphate and strontium polyacrylates;
vitamins such as Vitamins A, C and E;
plant extracts;
plant-derivable antioxidants such as flavonoid, catechin, polyphenol, and tannin compounds and mixtures thereof;
desensitising agents, e.g. potassium citrate, potassium chloride, potassium tartrate, potassium bicarbonate, potassium oxalate, potassium nitrate and strontium salts;
anti-calculus agents, e.g. alkali-metal pyrophosphates, hypophosphite-containing polymers, organic phosphonates and phosphocitrates etc.;
biomolecules, e.g. bacteriocins, antibodies, enzymes, etc.;
flavours, e.g. peppermint and spearmint oils;
proteinaceous materials such as collagen;

preservatives;
opacifying agents;
hyaluronic acid;
amino acids such as arginine;
colouring agents;
pH-adjusting agents;
sweetening agents;
pharmaceutically acceptable carriers, e.g. starch, sucrose, water or water/alcohol systems etc.;
surfactants, such as anionic, nonionic, cationic and zwitterionic or amphoteric surfactants;
Humectants such as glycerol, sorbitol, propyleneglycol, xylitol, lactitol etc.;
binders and thickeners such as sodium carboxymethyl-cellulose, hydroxyethyl cellulose (Natrosol®), xanthan gum, gum arabic etc. as well as synthetic polymers such as polyacrylates and carboxyvinyl polymers such as Carbopol®;
polymeric compounds which can enhance the delivery of active ingredients such as antimicrobial agents can also be included;
buffers and salts to buffer the pH and ionic strength of the oral care composition; and other optional ingredients that may be included are e.g. bleaching agents such as peroxy compounds e.g. potassium peroxydiphosphate, effervescing systems such as sodium bicarbonate/citric acid systems, colour change systems, and so on.

The invention will now be illustrated by the following non-limiting examples.

Examples of the invention are represented by a number, comparative Examples are illustrated by a letter.

## Examples

Sprays were produced as in Table 1

**Table 1**

| Ingredient | Example 1 | Example 2 | Example 3 | Example A |
|---|---|---|---|---|
| Sorbitol | 50.00 | 50.00 | 50.00 | 52.00 |
| Sodium Saccharin | 0.27 | 0.27 | 0.27 | 0.27 |
| Sodium hexametaphosphate | 0.50 | 0.50 | 0.50 | 0 |
| Pluronic F 127 | 1.50 | 1.50 | 1.50 | 0 |
| Blue covarine | 0.005 | 0.01 | 0.015 | 0 |
| Cremaphor | 2.50 | 2.50 | 2.50 | 2.50 |
| Flavour | 0.50 | 0.50 | 0.50 | 0.50 |
| Water | 44.73 | 44.73 | 44.73 | 44.73 |
| Total | 100 | 100 | 100 | 100 |

### Testing Method

Extracted teeth were cleaned with a silica-based toothpaste and placed in sterile human pooled saliva for 2 hours to allow a pellicle to form. Baseline tooth colour was measured using a colorimeter. The teeth were gently dabbed with a tissue to remove excess saliva and each tooth was then given 2 spray applications of either the antistain formulation (containing pluronic F127 and sodium hexametaphosphate) or the placebo formulation. The teeth were left for 10 seconds before before a colour measurement was taken (instant whitening effect) and then placed in a stain solution at 25°c (1:1:1 black coffee solution, black tea solution, red wine) with gentle agitation (75rpm) for 1.5 or 3 hours. The teeth were removed at each time point, rinsed in 50ml of water, the colour remeasured and then placed back in the stain.

The instant whitening effect is described in terms of changes in the tooth Whiteness Index (WIO) from baseline and the subsequent antistain in terms of ΔE from baseline. The results are shown in Table 2.

Total colour change (ΔE) from baseline and hence level of stain formation was calculated following different timepoints using:

$$\Delta E = \sqrt{[(L^*_0 - L^*_t)^2 + (a^*_0 - a^*_t)^2 + (b^*_0 - b^*_t)^2]}$$

Where $L^*_0$ and $L^*_t$ are L* values at baseline and time t respectively; $a^*_0$ and $a^*_t$ are a* values at baseline and time t respectively, and $b^*_0$ and $b^*_t$ are b* values at baseline and time t.

**Table 2**

| Formulation | Instant Whitening Effect Mean ΔWIO (s.e) | Stain Formation Mean ΔE (s.e) | |
|---|---|---|---|
| | 0.0hr | 1.5hr | 3.0hr |
| Example A | 0.1 (0.4) A | 6.0 (0.3) A | 7.9 (0.3) A |
| Example 1 | 4.8 (0.7) B | 2.7 (0.5) B | 2.9 (0.5) B |
| Example 2 | 6.7 (0.9) B | 2.4 (0.4) B | 1.6 (0.3) C |
| Example 3 | 12.3 (0.8) C | 2.9 (0.3) B | 3.5 (0.3) B |

**Results**

Mean ΔE (s.e.) values for each treatment group (n=15) after various time points in the staining solution are shown below in Table 2. The product differences were of statistical significance (Student's t-test, $p<0.05$) at each time point.

The data in the table 2 indicates that oral care compositions as per the invention (Example 1-3) provides vastly superior anti-staining efficacy (as evident by the low ΔE values) as compared to the comparative Example (Example A).

Values with different letters within each time point indicate statistically significant differences between treatment groups ($p<0.05$, ANOVA, Tukey-Kramer).

The data in Table 2 indicates that the addition of blue covarine at different levels to the spray formulation (Example 1) gives instant whitening benefits superior to the comparative formulation (Example A) as evident by the greater and significant change in WIO values. In addition, the blue covarine containing spray formulation provides vastly superior anti-staining efficacy (as evident by the significantly lower ΔE values) as compared to the comparative Example (Example A).

## Claims

1. An oral care composition comprising:

   (a) polymer having the structure

   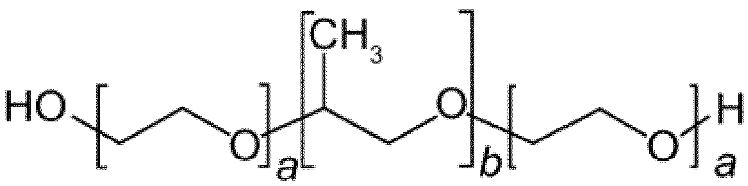

   in which a is independently selected from 2 to 130 and b is 15 to 67 and the molecular weight of the polymer is from 1,700 to 15,000 Da.
   (b) hexametaphosphate;
   (c) blue covarine;
   (d) an orally acceptable base;

   wherein the weight ratio of the polymer to hexametaphosphate is from 1:5 to 5:1.

2. A composition as claimed in claim 1 wherein the weight ratio of polymer to hexametaphosphate is from 1:4 to 4:1.

3. A composition as claimed in any one of the preceding claims wherein the molecular weight of the polymer is from 10,000 to 13,000 Da.

**4.** A composition as claimed in any one of the preceding claims comprising from 0.05 to 4wt%, preferably from 0,1 to 2 wt% of the total composition of polymer (a).

**5.** A composition as claimed in any one of the preceding claims comprising from 0.1 to 4 wt% of the total composition of hexametaphosphate.

**6.** A composition according to any preceding claim in which the total amount of polymer and hexametaphosphate is from 0.1 to 5wt% of the total composition.

**7.** A composition according to any preceding claim that is a spray composition.

**8.** A composition according to any of claims 1 to 6 that is a toothpaste.

**9.** A composition according to any preceding claim in which the level blue covarine is from 0.001 to 0.1 wt% of the total composition, preferably from 0.007 to 0.014 wt%.

**10.** A composition according to any preceding claim in which the weight ration of blue covarine to polymer (a) is from 5:100 to 1:1,000, preferably from 3:200 to 1:500.

**11.** A composition according to any preceding claim in which has a pH at 20°C from 6 to 9.

**12.** A composition according to any preceding claims wherein the orally acceptable base comprises a humectant, water, volatile alcohol or combinations thereof.

**13.** A composition as claimed in claim 10 wherein the humectant is a polyhydric alcohol preferably sorbitol or glycerol.

**14.** A non-therapeutic method of minimising or preventing the staining of teeth comprising the steps of applying the composition as claimed in any one of the preceding claims on to a tooth surface.

**15.** Cosmetic non-therapeutic use of a composition according to any one of claims 1 to 11 to mitigate the staining of teeth.

**Patentansprüche**

**1.** Mundpflegezusammensetzung, umfassend:

(a) Polymer mit der Struktur

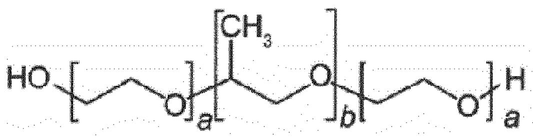,

in welcher a unabhängig aus 2 bis 130 ausgewählt ist und b 15 bis 67 beträgt und das Molekulargewicht des Polymers 1.700 bis 15.000 Da beträgt;
(b) Hexametaphosphat;
(c) Blue Covarine;
(d) eine oral akzeptable Basis;

wobei das Gewichtsverhältnis des Polymers zum Hexametaphosphat 1:5 bis 5:1 beträgt.

**2.** Zusammensetzung, wie im Anspruch 1 beansprucht, wobei das Gewichtsverhältnis des Polymers zum Hexametaphosphat 1:4 bis 4:1 beträgt.

**3.** Zusammensetzung, wie in irgendeinem der vorhergehenden Ansprüche beansprucht, wobei das Molekulargewicht des Polymers 10.000 bis 13.000 Da beträgt.

4. Zusammensetzung, wie in irgendeinem der vorhergehenden Ansprüche beansprucht, umfassend 0,05 bis 4 Gew.-%, vorzugsweise 0,1 bis 2 Gew.-% der gesamten Zusammensetzung an Polymer (a).

5. Zusammensetzung, wie in irgendeinem der vorhergehenden Ansprüche beansprucht, umfassend 0,1 bis 4 Gew.-% der gesamten Zusammensetzung an Hexametaphosphat.

6. Zusammensetzung nach einem vorhergehenden Anspruch, in welcher die Gesamtmenge des Polymers und des Hexametaphosphats 0,1 bis 5 Gew.-% der gesamten Zusammensetzung beträgt.

7. Zusammensetzung nach einem vorhergehenden Anspruch, die eine Sprayzusammensetzung ist.

8. Zusammensetzung nach einem der Ansprüche 1 bis 6, die eine Zahnpasta ist.

9. Zusammensetzung nach irgendeinem vorhergehenden Anspruch, in der der Anteil des Blue Covarine 0,001 bis 0,1 Gew.-% der gesamten Zusammensetzung, vorzugsweise 0,007 bis 0,014 Gew.-%, beträgt.

10. Zusammensetzung nach einem vorhergehenden Anspruch, in der das Gewichtsverhältnis des Blue Covarine zum Polymer (a) 5:100 bis 1:1.000, vorzugsweise 3:200 bis 1:500, beträgt.

11. Zusammensetzung nach einem vorhergehenden Anspruch, die bei 20°C einen pH-Wert von 6 bis 9 hat.

12. Zusammensetzung nach einem der vorhergehenden Ansprüche, wobei die oral akzeptable Basis ein Feuchthaltemittel, Wasser, flüchtigen Alkohol oder Kombinationen davon umfasst.

13. Zusammensetzung, wie im Anspruch 10 beansprucht, wobei das Feuchthaltemittel ein mehrwertiger Alkohol, vorzugsweise Sorbit oder Glycerin, ist.

14. Nicht-therapeutisches Verfahren zum Minimieren oder zum Verhindern des Verfärbens von Zähnen, umfassend die Schritte des Auftragens der Zusammensetzung, wie in irgendeinem der vorhergehenden Ansprüche beansprucht, auf eine Zahnoberfläche.

15. Kosmetische, nicht-therapeutische Verwendung einer Zusammensetzung nach irgendeinem der Ansprüche 1 bis 11 zum Abschwächen der Zahnverfärbung.

**Revendications**

1. Composition pour le soin oral comprenant :

(a) un polymère ayant la structure

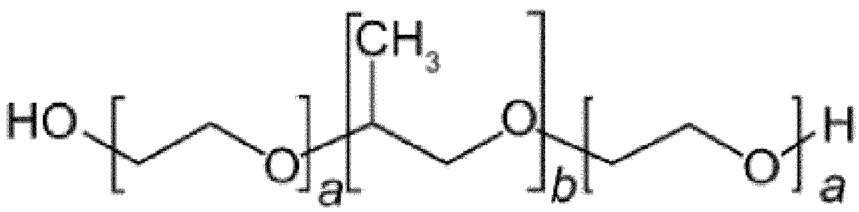

dans laquelle *a* est indépendamment choisi de 2 à 130 et *b* est de 15 à 67 et la masse moléculaire du polymère est de 1 700 à 15 000 Da
(b) de l'hexamétaphosphate ;
(c) de la covarine bleue ;
(d) une base oralement acceptable ;

dans laquelle le rapport en masse du polymère à l'hexamétaphosphate est de 1:5 à 5:1.

2. Composition selon la revendication 1, dans laquelle le rapport en masse de polymère à hexamétaphosphate est

de 1:4 à 4:1.

3. Composition selon l'une quelconque des revendications précédentes, dans laquelle la masse moléculaire du polymère est de 10 000 à 13 000 Da.

4. Composition selon l'une quelconque des revendications précédentes comprenant de 0,05 à 4 % en masse, de préférence de 0,1 à 2 % en masse de la composition totale de polymère (a).

5. Composition selon l'une quelconque des revendications précédentes comprenant de 0,1 à 4 % en masse de la composition totale d'hexamétaphosphate.

6. Composition selon l'une quelconque des revendications précédentes, dans laquelle la quantité totale de polymère et d'hexamétaphosphate est de 0,1 à 5 % en masse de la composition totale.

7. Composition selon l'une quelconque des revendications précédentes qui est une composition de pulvérisation.

8. Composition selon l'une quelconque des revendications 1 à 6 qui est un dentifrice.

9. Composition selon l'une quelconque des revendications précédentes, dans laquelle la teneur en covarine bleue est de 0,001 à 0,1 % en masse de la composition totale, de préférence de 0,007 à 0,014 % en masse.

10. Composition selon l'une quelconque des revendications précédentes, dans laquelle le rapport en masse de covarine bleue à polymère (a) est de 5:100 à 1:1 000, de préférence de 3:200 à 1:500.

11. Composition selon l'une quelconque des revendications précédentes, qui présente un pH à 20°C de 6 à 9.

12. Composition selon l'une quelconque des revendications précédentes, dans laquelle la base oralement acceptable comprend un humectant, de l'eau, un alcool volatil ou des combinaisons de ceux-ci.

13. Composition selon la revendication 10, dans laquelle l'humectant est un alcool polyvalent de préférence du sorbitol ou glycérol.

14. Procédé non-thérapeutique minimisant ou évitant la formation de taches sur les dents comprenant les étapes d'application de la composition selon l'une quelconque des revendications précédentes sur une surface de dent.

15. Utilisation non-thérapeutique cosmétique d'une composition selon l'une quelconque des revendications 1 à 11 pour mitiger la formation de taches sur les dents.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2014377194 A
- US 2003124065 A
- US 4370314 B